# EUROPEAN PATENT APPLICATION

(11) **EP 4 714 434 A1**
(43) Date of publication of application: **25.03.2026**
(21) Application number: 24811349.0
(22) Date of filing: 17.05.2024
(51) Int. Cl.: A61K 9/16, A61K 38/22, A61P 3/10, A61P 3/04, A61P 9/12, A61P 3/06, A61P 1/16, A61P 25/28, A61K 9/00

(54) **MICROSPHERE COMPRISING THYRAZEPATIDE, METHOD FOR PREPARING SAME, AND PHARMACEUTICAL COMPOSITION COMPRISING SAME**

(30) Priority: 19.05.2023 KR 20230065250
(71) Applicant: Aulbio Co., Ltd., Seongnam-si, Gyeonggi-do 13105 (KR)
(72) Inventor: KIM, Cherng Ju, Beaumont, California 92223 (US); AN, Tae Kun, Yongin-si, Gyeonggi-do 16990 (KR); KIM, A Ram, Seoul 05667 (KR); KIM, Seo A, Suwon-si, Gyeonggi-do 16509 (KR); PARK, Ji Won, Hwaseong-si, Gyeonggi-do 18266 (KR)
(74) Representative: dompatent
(86) International application number: PCT/KR2024/006716
(87) International publication number: WO 2024/242410

(57) **Abstract**

The present invention relates to: microspheres comprising tirzepatide or a pharmaceutically acceptable salt thereof, and a biocompatible polymer; and a method for preparing the same; and a pharmaceutical composition comprising the same.

The microspheres comprising tirzepatide according to the present invention can maintain an effective concentration of a drug in blood for a long period of time by exhibiting stable drug release for a long period of time, thereby extending a drug administration cycle, increasing drug compliance of a patient, and reducing side effects caused by rapid initial drug release.

## Description

### [Technical Field]

This application claims the benefit of priority based on Korean Patent Application No. 10-2023-0065250 filed May 19, 2023, the entire contents of which are incorporated as part of this specification.

The present invention relates to microspheres comprising tirzepatide, a method for preparing the same, and a pharmaceutical composition comprising the same.

### [Background Art]

T2D is the most common form of diabetes, accounting for approximately 90% of all diabetes. T2D is characterized by high blood sugar levels due to insulin-resistance. Current standard treatment for T2D includes diet and exercise, along with available oral and injectable glucose-lowering medications. Despite this, many patients with T2D remain inadequately controlled. Currently marketed incretin mimetics or dipeptidyl peptidase IV (DPP-IV) inhibitors utilize only a single established mechanism of action for glycemic control. Compounds for T2D that utilize a dual mechanism of action are needed.

GIP is a 42-amino acid gastrointestinal regulatory peptide that plays a physiologically important role in glucose homeostasis by stimulating insulin secretion from pancreatic beta cells in the presence of glucose and protecting pancreatic beta cells. GLP-1 is a 37-amino acid peptide that stimulates insulin secretion, protects pancreatic beta cells, and inhibits glucagon secretion, gastric emptying, and food intake, which induce weight loss. GIP and GLP-1 are known as incretins; incretin receptor signaling plays a physiologically relevant role in glucose homeostasis. In normal physiology, GIP and GLP-1 are secreted from the digestive tract after a meal, and these incretins promote physiological responses to food, including satiety, insulin secretion, and nutrient utilization. Patients with T2D exhibit impaired incretin responses.

GLP-1 analogue dosing has been shown to be limited by adverse effects, such as nausea and vomiting, and as a result, does not achieve full efficacy in glycemic control and weight loss. GIP alone has limited glucose-lowering capacity in humans with type 2 diabetes. Both natural GIP and GLP-1 are rapidly inactivated by the ubiquitous protease DPP IV, and therefore can only be used for short-term metabolic control.

Specific GIP analogues exhibiting both GIP and GLP-1 activity are disclosed in WO 2013/164483, WO 2014/192284, and WO 2011/119657. In addition, such GIP analogues as Mounjaro ^{®} and Zepbound ^{®} (tirzepatide) are commercially available.

Tirzepatide is often administered in the form of an injection due to several barriers, including enzymatic degradation in the gastrointestinal tract and intestinal mucosa, insufficient absorption from the intestinal mucosa, and first-pass metabolism in the liver.

Injectable drugs containing tirzepatide are formulated to be administered directly by patients (self-administration) for the continuous management of obesity or diabetes, so it is very important to manage pain and inflammatory reactions that may occur at the injection site.

Therefore, there is a need to develop a formulation that can maintain long-term efficacy with a single injection to facilitate the management of pain, inflammation, and the like, as described above, and to improve administration convenience. The currently available liquid formulation of tirzepatide (administered once a week) is easy to manufacture and administer, but increasing the dosage can lead to fatal side effects. Therefore, its use for the purpose of maintaining long-term efficacy is limited.

Recently, various formulation technologies have been employed to address these shortcomings and provide sustained and controlled release of pharmacologically active substances. Among these, injectable formulations, including long-acting microspheres, are injectable in which the pharmacologically active substance is encapsulated within a biocompatible polymer, and are designed to ensure uniform drug release from the microspheres upon subcutaneous or intramuscular injection.

In order for release formulations using such long-acting microspheres to actually lead to the effects of administration convenience and reduced dosage, it is necessary to develop a technology to contain a high dose of drug within the microspheres.

### [Prior Art Document]

### [Patent Document]

PCT Publication No. WO 2013/164483.

### [Disclosure]

### [Technical Problem]

The inventors of the present invention have conducted repeated research to solve the above-mentioned problems of the prior art and have developed microspheres in which tirzepatide is encapsulated at a high concentration, and which continuously release tirzepatide over a long period of time without an initial burst.

Therefore, the present invention aims to provide tirzepatide microspheres, which exhibit long-term stable drug release and maintain an effective blood concentration of the drug for a long period of time, thereby extending the drug administration cycle, increasing drug compliance of a patient, and reducing side effects caused by rapid initial drug release; a method for preparing the same; and a pharmaceutical composition comprising the same.

### [Technical Solution]

To solve the above problem, the present invention provides:
microspheres comprising tirzepatide or a pharmaceutically acceptable salt thereof, and a biocompatible polymer, wherein
the tirzepatide or a pharmaceutically acceptable salt thereof is contained in an amount of 9 wt% or more based on the total weight of the microspheres, and
the biocompatible polymer is polylactide-co-glycolide(PLGA) having an intrinsic viscosity of 0.15dL/g to 1.7dL/g, and a molar ratio of lactide to glycolide is 50:45 to 55.

In addition, the present invention provides a method for preparing microspheres, comprising the steps of:
(a) preparing a dispersed phase by dispersing tirzepatide or a pharmaceutically acceptable salt thereof and a biocompatible polymer in one or more solvents;
(b) adding the prepared dispersed phase to a continuous phase and stirring to form microspheres; and
(c) removing the solvent.

In addition, the present invention provides a method for preparing microspheres, comprising the steps of:
(1) preparing an inner aqueous phase (W₁) by dissolving or dispersing tirzepatide or a pharmaceutically acceptable salt thereof in a solvent;
(2) preparing an oil phase (O) by dissolving a biocompatible polymer in one or more solvents;
(3) preparing a water-in-oil (W₁/O) type emulsion by dispersing the inner aqueous phase (W₁) in the oil phase (O);
(4) forming microspheres by dispersing the water-in-oil (W₁/O) type emulsion into an outer continuous phase (W₂) to prepare a water-in-oil (W₁/O/W₂) type emulsion; and
(5) removing the solvent.

In addition, the present invention provides a pharmaceutical composition for preventing or treating diabetes, obesity, hypertension, hyperlipidemia, non-alcoholic steatohepatitis, metabolic steatohepatitis, cardiovascular disease, or degenerative neurological disease, comprising microspheres of the present invention and a pharmaceutically acceptable carrier.

### [Advantageous Effects]

The tirzepatide microspheres of the present invention exhibit stable drug release over a long period of time, thereby maintaining effective drug concentrations in the blood over a long period of time. Therefore, they provide the effects of extending the drug administration cycle, improving drug compliance of a patient, and alleviating side effects caused by rapid initial drug release.

Furthermore, the method for manufacturing tirzepatide microspheres of the present invention enables the efficient production of microspheres with excellent loading capacity and encapsulation rate. Furthermore, the pharmaceutical composition of the present invention, by including the tirzepatide microspheres, provides excellent effects in the treatment of diabetes, obesity, and the like.

### [Description of Drawings]

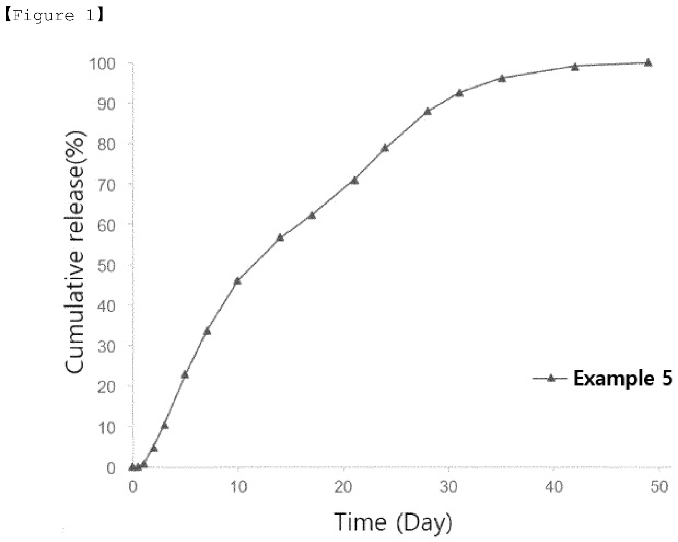

FIG. 1 is a graph showing the cumulative drug release rate over time after in-vivo administration of microspheres prepared in Example 5 of the present invention.

### [Best Mode]

Hereinafter, the present invention will be described in more detail.

All technical terms used herein, unless otherwise defined, have the same meaning as commonly understood by those skilled in the art. Furthermore, preferred methods or samples similar to or equivalent to those described herein are also included within the scope of the present invention. The contents of all publications cited herein are incorporated herein by reference in their entirety.

In the present invention, tirzepatide or a pharmaceutically acceptable salt thereof may be collectively referred to as tirzepatide.

The present invention relates to microspheres comprising tirzepatide or a pharmaceutically acceptable salt thereof, and a biocompatible polymer.

Currently, tirzepatide is marketed as a liquid injection (administered once a week). While liquid injections are easy to manufacture and administer, increasing the dosage can cause fatal side effects. Therefore, their use for maintaining long-term efficacy is limited.

The present invention provides tirzepatide microspheres to address the aforementioned problems of the prior art. The tirzepatide microspheres of the present invention exhibit stable drug release over a long period of time, thereby maintaining effective drug concentrations in the blood over a long period of time. Therefore, they can extend the drug administration cycle, improve drug compliance of a patient, and alleviate side effects caused by rapid initial drug release.

In addition, the present invention can show an excellent dose reduction effect by containing a high dose of tirzepatide in microspheres, thereby improving the convenience of administration to patients.

The tirzepatide is a GLP-1/GIP receptor agonist known as Ltyrosyl-2-methylalanyl-L-α-glutamylglycyl-L-threonyl-Lphenylalanyl-L-threonyl-L-seryl-L-α-aspartylL-tyrosyl-Lseryl-L-isoleucyl-2-methylalanyl-L-leucyl-L-α-aspartyl-Llysyl-L-isoleucyl-L-alanyl-Lglutaminyl-N6-[(22S)-22,42-dicarboxy-10,19,24-trioxo-3,6,12,15-tetraoxa-9,18,23-triazadotetracontan-1-oyl]-L-lysyl-L-alanyl-L-phenylalanyl-Lvalyl-L-glutaminyl-L-tryptophyl-L-leucylL-isoleucyl-Lalanylglycylglycyl-L-prolyl-L-seryl-L-serylglycyl-L-alanyl-Lprolyl-L-prolyl-L-prolyl-Lserinamide(CAS# 2023788-19-2).

The tirzepatide can be used for the prevention or treatment of diabetes (specific example: type 2 diabetes), obesity, preservation of beta-cell function, hypertension, hyperlipidemia, non-alcoholic steatohepatitis, metabolic steatohepatitis, cardiovascular disease, or degenerative neurological diseases such as Alzheimer's disease and Parkinson's disease.

As the pharmaceutically acceptable salt of the tirzepatide, any salt commonly used in this field may be used without limitation. The term "pharmaceutically acceptable salt" of the present invention refers to any organic or inorganic addition salt of tirzepatide at a concentration that is relatively non-toxic and harmless to the patient and has an effective effect, and the side effects caused by the salt do not reduce the beneficial effects of the active ingredient. Specific examples of the pharmaceutically acceptable salts include, but are not limited to, sodium, ammonium, magnesium, calcium, potassium, acetic acid, benzoic acid, hydroxynaphthoic acid, napadisylate, pamoate, fumaric acid, oxalic acid, citric acid, tartaric acid, hydrochloric acid, acetic acid, phosphoric acid, maleic acid, mesylic acid, edisylic acid, succinic acid, aspartic acid, pamoic acid, sulfuric acid, benzoic acid, besylic acid, and tosylic acid salt. The tirzepatide salts can be prepared, for example, by adding an acid to free tirzepatide to convert it into an acid addition salt, or by conventional techniques for converting one acid addition salt into another salt.

In the present invention, microspheres mean microspheres prepared using a biocompatible polymer in which tirzepatide or a pharmaceutically acceptable salt thereof is encapsulated, and these are simply referred to as tirzepatide-containing microspheres, tirzepatide microspheres, or microspheres. If tirzepatide or a pharmaceutically acceptable salt thereof is encapsulated in microspheres prepared using a biocompatible polymer, it may be included in the scope of the present invention depending on the molar ratio and intrinsic viscosity of the biocompatible polymer used.

The tirzepatide or a pharmaceutically acceptable salt thereof may be included in an amount of 9 wt% or more, 15 wt% or more, 20 wt% or more, 25 wt% or more, 30 wt% or more, 35 wt% or more, or 45 wt% or more, based on the total weight of the microspheres. If the drug contained in the microspheres is less than 9 wt%, it may be difficult to release the drug for more than 30 days. Additionally, the tirzepatide or a pharmaceutically acceptable salt thereof may be included in an amount of 60 wt% or less, 50 wt% or less, 40 wt% or less, 35 wt% or less, 30 wt% or less, 25 wt% or less, or 20 wt% or less, based on the total weight of the microspheres.

The tirzepatide or a pharmaceutically acceptable salt thereof may be included in a range by a combination of the above lower and upper limits.

In the present invention, the "biocompatible polymer" refers to a polymer whose safety is secured by ensuring that the polymer and its decomposition products do not cause cytotoxicity and inflammatory reactions in the body, and is also referred to simply as a polymer in this specification.

In the present invention, a biocompatible polymer having an intrinsic viscosity of 0.15 dL/g to 1.7 dL/g may be used, preferably a polymer having an intrinsic viscosity of 0.15 dL/g to 1.5 dL/g may be used, and more preferably a polymer having an intrinsic viscosity of 0.15 dL/g to 1.3 dL/g may be used.

If the intrinsic viscosity of the biocompatible polymer is less than 0.15 dL/g, the polymer may decompose too quickly, making it difficult to continuously release tirzepatide for a desired period of time. If it exceeds 1.7 dL/g, the polymer may decompose slowly, resulting in a small amount of tirzepatide released, and thus the drug may not be effective.

In the present invention, the biocompatible polymer may be included in an amount of 91 wt% or less based on the total weight of the microspheres. If the biocompatible polymer is included in an amount exceeding 91 wt%, the proportion of biocompatible polymer may be relatively high, resulting in a small amount of tirzepatide released, resulting in no or very slow efficacy. The biocompatible polymer may be included in an amount excluding the content range of the tirzepatide or a pharmaceutically acceptable salt thereof from the total weight of the microspheres. If the microspheres of the present invention further include other components (e.g., PVA, etc.) that can be added to the microspheres, the other components may be further included in an amount of 0.01 to 3 wt%, and in this case, the content of the biocompatible polymer may be reduced by the amount of the other components.

As the biocompatible polymer, polylactide-co-glycolide (PLGA) can be preferably used, and as the polylactide-co-glycolide (PLGA), at least one selected from copolymers having a molar ratio of lactide to glycolide of 50:45 to 55 or 50:48 to 52 can be more preferably used.

In one embodiment of the present invention, the microspheres can provide the encapsulated tirzepatide or a pharmaceutically acceptable salt thereof in a controlled or extended release form. The controlled or extended release form may be understood to have the same meaning as "sustained release," "controlled release," or "delayed release."

In one embodiment of the present invention, the microspheres may have a characteristic that the release of the encapsulated tirzepatide or a pharmaceutically acceptable salt thereof continues for more than two weeks in an in vitro environment.

In one embodiment of the present invention, tirzepatide or a pharmaceutically acceptable salt thereof contained in the microspheres may have an initial over-release (within 24 hours of initial burst) of 30 wt% or less, 28 wt% or less, 25 wt% or less, 20 wt% or less, 16 wt% or less, 13 wt% or less, 10 wt% or less, 7 wt% or less, 5 wt% or less, 3 wt% or less, or 2 wt% or less.

In addition, the microspheres of the present invention may be capable of continuously releasing tirzepatide or a pharmaceutically acceptable salt thereof for 14 days or more, preferably 25 days or more. Furthermore, the tirzepatide or a pharmaceutically acceptable salt thereof may be one continuously being released for 30 days or more, 40 days or more, or 50 days or more.

In one embodiment of the present invention, microspheres comprising tirzepatide or a pharmaceutically acceptable salt thereof may be prepared by a solvent evaporation or extraction method using an emulsion, and more preferably, an oil-in-water (O/W) type solvent evaporation method in which an O/W type emulsion comprising a biocompatible polymer, tirzepatide or a pharmaceutically acceptable salt thereof, and a dispersion solvent is prepared and the O/W type emulsion is coagulated into fine particles.

In order to prepare microspheres by a method of preparing the O/W type emulsion and coagulating it into polymer microparticles, first, an O/W type emulsion containing a biocompatible polymer, tirzepatide or a pharmaceutically acceptable salt thereof, and a dispersion solvent is prepared.

The O/W type emulsion can be prepared using a conventional method known in the art, and more specifically, it can be manufactured by adding a dispersed phase containing tirzepatide or a pharmaceutically acceptable salt thereof and a biocompatible polymer to a dispersion solvent.

Such microspheres containing tirzepatide or a pharmaceutically acceptable salt thereof can be prepared by coagulating an emulsion into microspheres using a solvent extraction method and/or a solvent evaporation method, or by coagulation through a process of ammonolysis with the addition of ammonia or hydrolysis with the addition of an acid or a base. A water-insoluble organic solvent that is converted into a water-soluble solvent by ammonolysis or hydrolysis reaction may be additionally included in the preparation of the emulsion.

In the case of the solvent evaporation method, it is not limited, but for example, a method described in U.S. Patent Nos. 5,271,945, 5,985,309 and 6,471,996, etc., that is, after dispersing or dissolving the drug in the organic solvent in which a polymer compound is dissolved in an organic solvent, emulsifying in a dispersion medium such as water to prepare an O/W type emulsion, and then diffusing the organic solvent in the emulsion into the dispersion medium and evaporating through the air/water interface, thereby forming polymer microspheres containing tirzepatide or a pharmaceutically acceptable salt thereof.

The solvent extraction method includes a conventional solvent extraction method used in the production of microspheres containing tirzepatide or a pharmaceutically acceptable salt thereof, such as effectively extracting the organic solvent in the emulsion droplets using a large amount of solubilizing solvent.

As a method of simultaneously applying the solvent evaporation method and the solvent extraction method, for example, the methods described in U.S. Patent Nos. 4,389,840, 4,530,840, 6,368,632, 6,544,559, and 6,572,894 can be applied.

The coagulation by the ammonolysis process refers to a method of coagulating fine particles by adding ammonia to an O/W type emulsion containing a water-insoluble organic solvent to induce ammonolysis, thereby converting the water-insoluble organic solvent into a water-soluble solvent, as described in, for example, Korean Patent No. 918092.

The coagulation by the hydrolysis process refers to a method of coagulating fine particles by adding a base such as NaOH, LiOH, or KOH, or an acid solution such as HCl or H₂SO₄ to an O/W type emulsion containing a water-insoluble organic solvent, thereby inducing hydrolysis, which is a type of hydrolysis reaction of ester, and converting the water-insoluble organic solvent into a water-soluble solvent, as described in, for example, Korean Patent Application Nos. 2009-109809 and 2010-70407.

In one embodiment of the present invention, the microspheres may be W₁/O/W₂ (water-in-oil-in-water) type microspheres prepared by preparing a W₁/O (water-in-oil) type emulsion including an inner aqueous phase (W₁) in which tirzepatide or a pharmaceutically acceptable salt thereof is dispersed or dissolved in an aqueous solvent; and an oil phase (O) in which a biocompatible polymer is dissolved in a non-aqueous solvent; and dispersing the same in an outer aqueous phase (W₂). In this case, the microspheres may be prepared by a double emulsification evaporation method.

In the present invention, microspheres containing tirzepatide or a pharmaceutically acceptable salt thereof can be prepared by various microsphere preparing methods known in the art (e.g., O/W type, O/O type or W/O/W type solvent evaporation method or solvent extraction method, microsphere preparing method by spray drying, microsphere preparing method by phase separation, etc.) .

In order to prepare microspheres by preparing the W/O/W type emulsion and coagulating it into polymer microparticles, first, a W/O/W type emulsion containing a biocompatible polymer, tirzepatide or a pharmaceutically acceptable salt thereof, and a dispersion solvent is prepared.

At this time, the W/O/W type emulsion can be manufactured using a conventional method known in the art, and more specifically, it can be prepared by adding a dispersed phase containing tirzepatide or a pharmaceutically acceptable salt thereof and a biocompatible polymer to a dispersion solvent.

These tirzepatide or a pharmaceutically acceptable salt thereof-containing microspheres can be prepared by coagulating an emulsion into microspheres by a solvent extraction method and/or a solvent evaporation method, or by coagulation through a process of ammonolysis with the addition of ammonia or hydrolysis with the addition of an acid or base. A water-insoluble organic solvent that is converted into a water-soluble solvent by the ammonolysis or hydrolysis reaction can be additionally included during the preparation of the emulsion.

In the case of the solvent evaporation method, it is not limited to, but for example, a method described in U.S. Patent Nos. 5,271,945, 5,985,309, and 6,471,996, that is, after dispersing or dissolving the drug in the organic solvent in which a polymer compound is dissolved in an organic solvent, emulsifying in a dispersion medium such as water to prepare a W/O/W type emulsion, and then diffusing the organic solvent in the emulsion into the dispersion medium and evaporating through the air/water interface, thereby forming polymer microspheres containing tirzepatide or a pharmaceutically acceptable salt thereof.

The solvent extraction method includes a conventional solvent extraction method used in the preparation of microspheres containing tirzepatide or a pharmaceutically acceptable salt thereof, such as effectively extracting the organic solvent in the emulsion droplets using a large amount of solubilizing solvent.

As a method of simultaneously applying the solvent evaporation method and the solvent extraction method, for example, the methods described in U.S. Patent Nos. 4,389,840, 4,530,840, 6,368,632, 6,544,559, and 6,572,894 can be applied.

The coagulation by the ammonolysis process refers to a method of coagulating fine particles by adding ammonia to an O/W type emulsion containing a water-insoluble organic solvent to induce ammonolysis, thereby converting the water-insoluble organic solvent into a water-soluble solvent, as described in, for example, Korean Patent No. 918092.

The coagulation by the hydrolysis process refers to a method of coagulating fine particles by adding a base such as NaOH, LiOH, or KOH, or an acid solution such as HCl or H₂SO₄ to a W/O/W type emulsion containing a water-insoluble organic solvent, thereby inducing hydrolysis, which is a type of hydrolysis reaction of ester, and converting the water-insoluble organic solvent into a water-soluble solvent, as described in, for example, Korean Patent Application Nos. 2009-109809 and 2010-70407.

In addition, the present invention provides a method for preparing microspheres, comprising the steps of:
(a) preparing a dispersed phase by dispersing tirzepatide or a pharmaceutically acceptable salt thereof and a biocompatible polymer in one or more solvents;
(b) adding the prepared dispersed phase to a continuous phase and stirring to form microspheres; and
(c) removing the solvent.

All of the contents described above regarding microspheres can be applied to the method for preparing microspheres of the present invention. Therefore, any redundant descriptions will be omitted below.

In the step (a), the weight ratio of the biocompatible polymer to tirzepatide or a pharmaceutically acceptable salt thereof may be 9 times or less.

In one embodiment of the present invention, a solvent for dissolving the biocompatible polymer or for preparing it into a dispersed phase is not particularly limited in type, and for example, dimethyl sulfoxide, dichloromethane, or a mixed solvent thereof may be used. In particular, dichloromethane may be preferably used.

In one embodiment of the present invention, tirzepatide or a pharmaceutically acceptable salt thereof and a biocompatible polymer may be sequentially dissolved and mixed in the same container. In this case, for example, dimethyl sulfoxide and dichloromethane may be used as a solvent.

The step (b) is a step of solidifying microspheres by dispersing the dispersed phase prepared in the step (a) into the outer continuous phase to prepare an emulsion solution (O/W).

In the step (b), a hydrophilic polymer may be included as a surfactant, and the type thereof is not particularly limited, and any one that can help the dispersed phase including tirzepatide or a pharmaceutically acceptable salt thereof and a biocompatible polymer to form a stable droplet of the dispersed phase within the outer continuous phase may be used.

The hydrophilic polymer may preferably be selected from the group consisting of methylcellulose, polyvinylpyrrolidone, carboxymethylcellulose, lecithin, gelatin, polyvinyl alcohol, polyoxyethylene-polyoxypropylene block copolymer, polyoxyethylene sorbitan fatty acid ester, polyoxyethylene castor oil derivative, and mixtures thereof, and polyvinyl alcohol may be preferably used.

In the step (b), the outer continuous phase may be a hydrophilic polymer aqueous solution of 0.1 to 6% (w/v), preferably 0.1 to 4% (w/v), wherein the weight average molecular weight of the hydrophilic polymer may be 7,000 to 40,000, and the degree of hydrolysis may be 80 to 90%.

In the step (b), the dispersed phase containing tirzepatide or a pharmaceutically acceptable salt thereof and a biocompatible polymer prepared in the step (a) is added to the outer continuous phase containing the hydrophilic polymer by a drop-by-drop method or by a method of using an in-line mixer, and stirred vigorously to prepare an emulsion solution (O/W).

Afterwards, in the step (c), the solvent is removed, and after conventional filtration and washing, the desired microspheres can be obtained. That is, a step of washing the obtained microspheres using an organic solvent such as ethanol may be included to enhance the initial release inhibition effect, if necessary.

In addition, the present invention provides a method for preparing microspheres, comprising the steps of:
(1) preparing an inner aqueous phase (W₁) by dissolving or dispersing tirzepatide or a pharmaceutically acceptable salt thereof in a solvent;
(2) preparing an oil phase (O) by dissolving a biocompatible polymer in one or more solvents;
(3) preparing a water-in-oil (W₁/O) type emulsion by dispersing the inner aqueous phase (W₁) in the oil phase (O);
(4) forming microspheres by adding the water-in-oil (W₁/O) type emulsion into an outer continuous phase (W₂) and dispersing to prepare a water-in-oil (W₁/O/W₂) type emulsion; and
(5) removing the solvent.

The method for manufacturing microspheres of the present invention may be applied to all of the above-described microspheres and methods for manufacturing microspheres that are compatible with the manufacturing method (type of biocompatible polymer, intrinsic viscosity thereof, etc.). Therefore, any redundant descriptions will be omitted below.

The step (1) may be, for example, a step of preparing a water phase (W₁) by dissolving tirzepatide or a pharmaceutically acceptable salt thereof in distilled water.

In the step (2), the type of solvent is not particularly limited, and for example, dimethyl sulfoxide, dichloromethane, or a mixed solvent thereof may be used. In particular, dichloromethane may be preferably used.

In the step (3), the weight ratio of the biocompatible polymer to tirzepatide or a pharmaceutically acceptable salt thereof may be 9 times or less.

The step (3) is a step of preparing a water-in-oil (W₁/O) type emulsion by dispersing the inner aqueous phase (W₁) prepared in the step (1) into the oil phase (O) prepared in the step (2).

In the step (3), the weight ratio of the oil phase (O) to the inner aqueous phase (W₁) may be 1:10 or less.

The step (4) is a step of dispersing the water-in-oil (W₁/O) type dispersed phase prepared in the step (3) into the outer continuous phase (W₂) to prepare a water-in-oil-in-water (W₁/O/W₂) type emulsion solution and solidifying microspheres.

In the step (4), the outer continuous phase (W₂) may contain a hydrophilic polymer as a surfactant, and the type thereof is not particularly limited, and any one that can help the dispersed phase containing tirzepatide or a pharmaceutically acceptable salt thereof and a biocompatible polymer to form a stable droplet of the dispersed phase within the outer continuous phase may be used.

The hydrophilic polymer may preferably be selected from the group consisting of methylcellulose, polyvinylpyrrolidone, carboxymethylcellulose, lecithin, gelatin, polyvinyl alcohol, polyoxyethylene-polyoxypropylene block copolymer, polyoxyethylene sorbitan fatty acid ester, polyoxyethylene castor oil derivative, and mixtures thereof, and polyvinyl alcohol may be preferably used.

In the step (4), the outer continuous phase may be a hydrophilic polymer aqueous solution of 0.1 to 6% (w/v), preferably 0.1 to 4% (w/v), and at this time, the weight average molecular weight of the hydrophilic polymer may be 7,000 to 40,000, and the degree of hydrolysis may be 80 to 90%.

In the step (4), the dispersed phase (W₁/O) containing tirzepatide or a pharmaceutically acceptable salt thereof and a biocompatible polymer prepared in the step (3) is added to the outer continuous phase containing the hydrophilic polymer by a drop-by-drop method or by a method of using an in-line mixer, and stirred vigorously to prepare a water-in-oil-in-water (W₁/O/W₂) type emulsion solution.

After the solvent is removed in the step (5), the desired microspheres can be obtained after conventional filtration and washing. That is, a step of washing the obtained microspheres with an organic solvent such as ethanol may be included to enhance the initial release inhibition effect, if necessary.

In addition, the present invention
provides a pharmaceutical composition for preventing or treating diabetes, obesity, hypertension, hyperlipidemia, non-alcoholic steatohepatitis, metabolic steatohepatitis, cardiovascular disease, or degenerative neurological disease, comprising the microspheres and a pharmaceutically acceptable carrier.

The pharmaceutical composition according to the present invention can be formulated for parenteral administration.

The pharmaceutical composition for parenteral administration may contain the microspheres alone or may further comprise a pharmaceutically acceptable carrier for parenteral administration, which may be conventionally added to the pharmaceutical composition. Furthermore, it may further contain an excipient or diluent. The carrier include all types of solvents, dispersion media, oil-in-water or water-in-oil emulsions, aqueous compositions, liposomes, microbeads, and microsomes.

The carrier for parenteral administration may include water, suitable oils, saline, aqueous glucose, and glycol, and may further include stabilizers and preservatives.

Suitable stabilizers include antioxidants such as sodium bisulfite, sodium sulfite, or ascorbic acid. Suitable preservatives include benzalkonium chloride, methyl- or propylparaben, and chlorobutanol.

The pharmaceutical composition of the present invention may further include, in addition to the above ingredients, lubricants, wetting agents, sweeteners, flavoring agents, emulsifiers, suspending agents, etc. Other pharmaceutically acceptable carriers and preparations may be referred to as described in the following literature (Remington's Pharmaceutical Sciences, 19th ed., Mack Publishing Company, Easton, PA, 1995).

The pharmaceutical composition of the present invention may be administered to a patient (e.g., a human in need of such a drug) or other animals parenterally, for example, but not limited to, by intravenous, intramuscular, intraarterial, intramedullary, intrathecal, intracardiac, transdermal, subcutaneous, intraperitoneal, intranasal, enteral, intravaginal, intrapulmonary, suppository, topical, sublingual or rectal administration.

In the present invention, 'treatment' means any act of improving or beneficially altering an (acute or chronic) disease, disorder, and symptoms resulting therefrom by administering a pharmaceutical composition. In addition, the above 'treatment' broadly includes the meaning of 'prevention,' whereby 'prevention' means any act of suppressing or delaying the onset of a disease and symptoms resulting therefrom by administering a pharmaceutical preparation. The above 'treatment' includes, for example, the obstruction, alleviation, improvement, cessation, suppression, delay, and reversal of the progression of an (acute or chronic) disease, disorder, and symptoms resulting therefrom.

A preferred total dosage of the pharmaceutical composition or agent of the present invention may be about 0.001 mg to 2,000 mg based on tirzepatide. However, the dosage, frequency, and duration of the pharmaceutical composition will vary depending on factors such as the nature and severity of the condition to be treated, the age and general health of the subject (host), and the tolerance of the subject (host) to the active ingredient. Taking these points into account, a person of ordinary skill in the art will be able to determine an appropriate effective dosage of the composition of the present invention. The pharmaceutical composition according to the present invention is not particularly limited in its formulation, administration route, and administration method as long as it exhibits the effects of the present invention.

Hereinafter, the present invention will be described in detail using examples to specifically describe the invention. However, the examples according to the present invention may be modified in various different forms, and the scope of the present invention should not be construed as being limited to the examples described below. The examples of the present invention are provided to more fully describe the present invention to those of average skill in the art.

### Example 1: Preparation of tirzepatide microspheres

### <Preparation of continuous phase>

1,000 mL of distilled water and 10 g of PVA were weighed and added to the reactor, and then stirred at 1,000 rpm using a homogenizer (Overhead (IKA)) to prepare a 1% (w/v) PVA aqueous solution.

### <Preparation of dispersed phase>

0.09 g of tirzepatide and 2 ml of dimethyl sulfoxide were weighed and added to an 8 mL vial, and stirred at 150 rpm to dissolve.

After the solution dissolved, 0.8 g of PLGA (intrinsic viscosity of 0.15 to 1.7 dL/g) and 2 ml of dichloromethane were added, and stirred at 150 rpm to dissolve.

### <Recovery of microspheres>

After the above process was completed, the dispersed phase was immediately taken with a syringe and then placed in a 1 L reactor into which the continuous phase had been previously added, while stirring with a homogenizer for 7 seconds. The microspheres were then solidified for 24 hours. After solidification was completed, the microspheres were obtained by filtering through a 5 µm filter. The obtained microspheres were then freeze-dried in a freeze dryer for more than 20 hours.

### Example 2: Preparation of tirzepatide microspheres

### <Preparation of continuous phase>

1,000 mL of distilled water and 10 g of PVA were weighed and added to the reactor, and then stirred at 1,000 rpm using a homogenizer (Overhead (IKA)) to prepare a 1% (w/v) PVA aqueous solution.

### <Preparation of dispersed phase>

0.08g of tirzepatide and 2 ml of dimethyl sulfoxide were weighed and added to an 8 mL vial, and stirred at 150 rpm to dissolve.

After the solution dissolved, 0.7 g of PLGA (intrinsic viscosity of 0.15 to 1.7 dL/g) and 2 ml of dichloromethane were added, and stirred at 150 rpm to dissolve.

### <Recovery of microspheres>

After the above process was completed, the dispersed phase was immediately taken with a syringe and then placed in a 1 L reactor into which the continuous phase had been previously added, while stirring with a homogenizer for 7 seconds. The microspheres were then solidified for 24 hours. After solidification was completed, the microspheres were obtained by filtering through a 5 µm filter. The obtained microspheres were then freeze-dried in a freeze dryer for more than 20 hours.

### Example 3: Preparation of tirzepatide microspheres

### <Preparation of continuous phase>

1,000 mL of distilled water and 10 g of PVA were weighed and added to the reactor, and then stirred at 1,000 rpm using a homogenizer (Overhead (IKA)) to prepare a 1% (w/v) PVA aqueous solution.

### <Preparation of dispersed phase>

0.175g of tirzepatide and 2 ml of dimethyl sulfoxide were weighed and added to an 8 mL vial, and stirred at 150 rpm to dissolve.

After the solution dissolved, 0.7g of PLGA (intrinsic viscosity of 0.15 to 1.7 dL/g) and 2 ml of dichloromethane were added, and stirred at 150 rpm to dissolve.

### <Recovery of microspheres>

After the above process was completed, the dispersed phase was immediately taken with a syringe and then placed in a 1 L reactor into which the continuous phase had been previously added, while stirring with a homogenizer for 7 seconds. The microspheres were then solidified for 24 hours. After solidification was completed, the microspheres were obtained by filtering through a 5 µm filter. The obtained microspheres were then freeze-dried in a freeze dryer for more than 20 hours.

### Example 4: Preparation of tirzepatide microspheres

### <Preparation of continuous phase>

900 ml of distilled water was added to the reactor, 4.5 g of PVA and 4.5 g of NaCl were weighed and added, and then stirred at 1,000 rpm using a homogenizer (Overhead (IKA)) to prepare a 0.5% (w/v) PVA aqueous solution.

### <Preparation of tirzepatide solution>

0.3 g of tirzepatide and 1.0 ml of distilled water were weighed and added to a 20 ml vial, and stirred at 150 rpm to dissolve.

### <Preparation of PLGA solution>

1.2 g of PLGA (intrinsic viscosity of 0.15 dL/g to 1.7 dL/g) was added into 3 ml of dichloromethane and stirred at 300 rpm to dissolve.

### <Emulsification>

The PLGA solution prepared above was taken with a syringe and added to the tirzepatide solution, and then stirred at 20,000 rpm for 1 minute using a homogenizer (IKA).

### <Recovery of microspheres>

After the above emulsification process was completed, the solution was immediately taken into a syringe and placed in a 1 L reactor into which the continuous phase had been previously added, while stirring with a homogenizer for 60 seconds. The microspheres were then solidified for 20 hours. After solidification was completed, the microspheres (W/O/W type) were obtained by filtering through a 5 µm filter. The obtained microspheres were then freeze-dried in a freeze dryer for more than 20 hours.

### Example 5: Preparation of tirzepatide microspheres

### <Preparation of continuous phase>

900 ml of distilled water was added to the reactor, 4.5 g of PVA and 4.5 g of NaCl were weighed and added, and then stirred at 1,000 rpm using a homogenizer (Overhead (IKA)) to prepare a 0.5% (w/v) PVA aqueous solution.

### <Preparation of tirzepatide solution>

0.386 g of tirzepatide and 1.5 ml of distilled water were weighed and added to a 20 ml vial, and stirred at 150 rpm to dissolve.

### <Preparation of PLGA solution>

0.9 g of PLGA (intrinsic viscosity of 0.15 dL/g to 1.7 dL/g) was added into 3 ml of dichloromethane and stirred at 300 rpm to dissolve.

### <Emulsification>

The PLGA solution prepared above was taken with a syringe and added into the tirzepatide solution, and then stirred at 20,000 rpm for 1 minute using a homogenizer (IKA).

### <Recovery of microspheres>

After the above emulsification process was completed, the solution was immediately taken into a syringe and added in a 1 L reactor into which the continuous phase had been previously added, while stirring with a homogenizer for 60 seconds. The microspheres were then solidified for 20 hours. After solidification was completed, microspheres (W/O/W type) were obtained by filtering through a 5 µm filter. The obtained microspheres were then freeze-dried in a freeze dryer for more than 20 hours.

### Example 6: Preparation of tirzepatide microspheres

### <Preparation of continuous phase>

900 ml of distilled water was added to the reactor, 4.5 g of PVA and 4.5 g of NaCl were weighed and added, and then stirred at 1,000 rpm using a homogenizer (Overhead (IKA)) to prepare a 0.5% (w/v) PVA aqueous solution.

### <Preparation of tirzepatide solution>

0.324 g of tirzepatide and 1 ml of distilled water were weighed and added to a 20 ml vial, and stirred at 150 rpm to dissolve.

### <Preparation of PLGA solution>

0.6 g of PLGA (intrinsic viscosity of 0.15 dL/g to 1.7 dL/g) was added into 2 ml of dichloromethane and stirred at 300 rpm to dissolve.

### <Emulsification>

The PLGA solution prepared above was taken with a syringe and added into the tirzepatide solution, and then stirred at 20,000 rpm for 1 minute using a homogenizer (IKA).

### <Recovery of microspheres>

After the above emulsification process was completed, the solution was immediately taken into a syringe and added in a 1 L reactor into which the continuous phase had been previously added, while stirring with a homogenizer for 60 seconds. The microspheres were then solidified for 20 hours. After solidification was completed, microspheres (W/O/W type) were obtained by filtering through a 5 µm filter. The obtained microspheres were then freeze-dried in a freeze dryer for more than 20 hours.

### Example 7: Preparation of tirzepatide microspheres

### <Preparation of continuous phase>

900 ml of distilled water was added to the reactor, 4.5 g of PVA and 4.5 g of NaCl were weighed and added, and then stirred at 1,000 rpm using a homogenizer (Overhead (IKA)) to prepare a 0.5% (w/v) PVA aqueous solution.

### <Preparation of tirzepatide solution>

0.269 g of tirzepatide and 1 ml of distilled water were weighed and added to a 20 ml vial, and stirred at 150 rpm to dissolve.

### <Preparation of PLGA solution>

0.5 g of PLGA (intrinsic viscosity of 0.15 dL/g to 1.7 dL/g) was added into 2 ml of dichloromethane and stirred at 300 rpm to dissolve.

### <Emulsification>

The PLGA solution prepared above was taken with a syringe and added into the tirzepatide solution, and then stirred at 20,000 rpm for 1 minute using a homogenizer (IKA).

### <Recovery of microspheres>

After the above emulsification process was completed, the solution was immediately taken into a syringe and added in a 1 L reactor into which the continuous phase had been previously added, while stirring with a homogenizer for 60 seconds. The microspheres were then solidified for 20 hours. After solidification was completed, microspheres (W/O/W type) were obtained by filtering through a 5 µm filter. The obtained microspheres were then freeze-dried in a freeze dryer for more than 20 hours.

### Example 8: Preparation of tirzepatide microspheres

### <Preparation of continuous phase>

900 ml of distilled water was added to the reactor, 4.5 g of PVA and 4.5 g of NaCl were weighed and added, and then stirred at 1,000 rpm using a homogenizer (Overhead (IKA)) to prepare a 0.5% (w/v) PVA aqueous solution.

### <Preparation of tirzepatide solution>

0.4 g of tirzepatide and 1 ml of distilled water were weighed and added to a 20 ml vial, and stirred at 150 rpm to dissolve.

### <Preparation of PLGA solution>

0.6 g of PLGA (intrinsic viscosity of 0.15 dL/g to 1.7 dL/g) was added into 2 ml of dichloromethane and stirred at 300 rpm to dissolve.

### <Emulsification>

The PLGA solution prepared above was taken with a syringe and added into the tirzepatide solution, and then stirred at 20,000 rpm for 1 minute using a homogenizer (IKA).

### <Recovery of microspheres>

After the above emulsification process was completed, the solution was immediately taken into a syringe and placed in a 1 L reactor into which the continuous phase had been previously added, while stirring with a homogenizer for 60 seconds. The microspheres were then solidified for 20 hours. After solidification was completed, microspheres (W/O/W type) were obtained by filtering through a 5 µm filter. The obtained microspheres were then freeze-dried in a freeze dryer for more than 20 hours.

### Comparative Example 1: Preparation of tirzepatide microspheres

### <Preparation of continuous phase>

900 ml of distilled water, 4.5 g of PVA (manufactured by Sigma company), and 4.5 g of NaCl were weighed and added to the reactor, and then stirred at 1,000 rpm using a homogenizer (Overhead (IKA)) to prepare a 0.5% (w/v) PVA aqueous solution.

### <Preparation of tirzepatide solution>

0.24 g of tirzepatide and 2.4 ml of distilled water were weighed and added to a 20 ml vial, and stirred at 150 rpm to dissolve.

### <Preparation of PLGA solution>

4.8 g of PLGA (intrinsic viscosity of 0.10 dL/g to 0.31 dL/g) and PLGA (intrinsic viscosity of 0.32 dL/g to 0.74 dL/g) was add into 12 ml of dichloromethane and stirred at 300 rpm to dissolve.

### <Emulsification>

The PLGA solution prepared above was taken with a syringe and added to the tirzepatide solution, and then stirred at 20,000 rpm for 1 minute using a homogenizer (IKA).

### <Recovery of microspheres>

After the emulsification process was completed, the solution was immediately taken into a syringe and placed in a 1 L reactor into which the continuous phase had been previously added, while stirring with a homogenizer for 60 seconds at 3,500 rpm. The microspheres were then solidified for 24 hours. After solidification was completed, microspheres (W/O/W type) were obtained by filtering through a 5 µm filter. The obtained microspheres were then freeze-dried in a freeze dryer for more than 20 hours.

### Comparative Example 2: Preparation of tirzepatide microspheres

### <Preparation of continuous phase>

900 ml of distilled water was added to the reactor, 4.5 g of PVA and 4.5 g of NaCl were weighed and added, and then stirred at 1,000 rpm using a homogenizer (Overhead (IKA)) to prepare a 0.5% (w/v) PVA aqueous solution.

### <Preparation of tirzepatide solution>

0.323 g of tirzepatide and 1 ml of distilled water were weighed and added to a 20 ml vial, and stirred at 150 rpm to dissolve.

### <Preparation of PLGA solution>

0.6 g of PLGA (intrinsic viscosity of 0.32 dL/g to 0.44 dL/g) was added into 2 ml of dichloromethane and stirred at 300 rpm to dissolve.

### <Emulsification>

The PLGA solution prepared above was taken with a syringe and added to the tirzepatide solution, and then stirred at 20,000 rpm for 1 minute using a homogenizer (IKA).

### <Recovery of microspheres>

After the above emulsification process was completed, the solution was immediately taken into a syringe and placed in a 1 L reactor into which the continuous phase had been previously added, while stirring with a homogenizer for 60 seconds. The microspheres were then solidified for 20 hours. After solidification was completed, microspheres (W/O/W type) were obtained by filtering through a 5 µm filter. The obtained microspheres were then freeze-dried in a freeze dryer for more than 20 hours.

### Comparative Example 3: Preparation of tirzepatide microspheres

### <Preparation of continuous phase>

900 ml of distilled water was added to the reactor, 4.5 g of PVA and 4.5 g of NaCl were weighed and added, and then stirred at 1,000 rpm using a homogenizer (Overhead (IKA)) to prepare a 0.5% (w/v) PVA aqueous solution.

### <Preparation of tirzepatide solution>

0.41 g of tirzepatide and 1 ml of distilled water were weighed and added to a 20 ml vial, and stirred at 150 rpm to dissolve.

### <Preparation of PLGA solution>

0.6 g of PLGA (intrinsic viscosity of 0.32 dL/g to 0.44 dL/g) was added into 2 ml of dichloromethane and stirred at 300 rpm to dissolve.

### <Emulsification>

The PLGA solution prepared above was taken with a syringe and added to the tirzepatide solution, and then stirred at 20,000 rpm for 1 minute using a homogenizer (IKA).

### <Recovery of microspheres>

After the above emulsification process was completed, the solution was immediately taken into a syringe and placed in a 1 L reactor into which the continuous phase had been previously added, while stirring with a homogenizer for 60 seconds. The microspheres were then solidified for 20 hours. After solidification was completed, microspheres (W/O/W type) were obtained by filtering through a 5 µm filter. The obtained microspheres were then freeze-dried in a freeze dryer for more than 20 hours.

### Experimental Example 1: Confirmation of loading rate of tirzepatide microspheres

The microspheres prepared in the Examples 1 to 8 and Comparative Example 1 were placed in a 20 ml volumetric flask and completely dissolved in acetonitrile containing 0.1% TFA (Trifluoroacetic Acid). The solution was adjusted to the mark with distilled water containing 0.1% TFA (Trifluoroacetic Acid) and filtered through a 0.45 um syringe filter. The solution was detected using HPLC with a UV detector.

The results of the above experiment are shown in Table 1 below.

**[Table 1]**

| | Theoretical loading rate(wt%) | Actual loading rate (wt%) |
|---|---|---|
| Example1 | 10 | 9.35 |
| Example2 | 10 | 9.50 |
| Example3 | 20 | 17.22 |
| Example4 | 20 | 20.86 |
| Example 5 | 30 | 30.83 |
| Example 6 | 35 | 36.32 |
| Example 7 | 35 | 35.60 |
| Example 8 | 40 | 40.35 |
| Comparative Example 1 | 4.76 | 4.95 |
| Comparative Example 2 | 35 | 35.34 |
| Comparative Example 3 | 40 | 38.87 |

### Experimental Example 2: In vitro release rate evaluation

The microspheres prepared in Examples 1 to 8 were placed in vials, a pH 7.4 PBS solution was added, and stirred at 100 rpm while maintaining at 37°C. To measure the release amount over a given period of time, the supernatant was filtered through a 0.45 um RC filter and used as a test solution, and the new release solution was then placed in the vial. The test solution was detected using HPLC with a UV-visible spectrophotometer.

The experimental results are shown in Table 2.

**[Table 2]**

| | Initial burst(wt%) | Release at day 10 (wt%) |
|---|---|---|
| Example1 | 2.33 | 49.55 |
| Example2 | 0.26 | 11.82 |
| Example3 | 12.81 | 33.21 |
| Example4 | 1.46 | 41.61 |
| Example 5 | 1.47 | 47.62 |
| Example 6 | 6.59 | 66.37 |
| Example 7 | 11.78 | 79.14 |
| Example 8 | 16.17 | 73.67 |
| Comparative Example 2 | 5.00 | 87.06 |
| Comparative Example 3 | 6.28 | 100.36 |

As confirmed in Table 2, the microspheres of Examples 1 to 8, in which tirzepatide is included in an amount of 9 wt% or more based on the total weight of the microspheres and the weight of the biocompatible polymer with respect to tirzepatide is included in an amount of 91 wt% or less, exhibit an initial burst of 20% or less within 24 hours, and long-term sustained release is possible. In addition, the microspheres of Comparative Examples 2 and 3, in which the biocompatible polymer is prepared in a range beyond the range of a copolymer having a molar ratio of lactide to glycolide of polylactide-co-glycolide (PLGA) of 50:45 to 55, exhibit a release rate of 85% or more on the 10th day, and long-term sustained release is difficult.

Therefore, it is preferable to use a copolymer of polylactide-co-glycolide (PLGA) having a molar ratio of lactide to glycolide of 50:45 to 55 as the biocompatible polymer.

### Experimental Example 3: In-vivo pharmacokinetic study using beagles

After subcutaneously injecting the tirzepatide microsphere formulation (Example 5) into a beagle, blood samples were collected at designated times and the blood tirzepatide concentration was measured using LC-MS/MS.

The cumulative drug release rate from the above experiment results is shown in Figure 1.

As shown in FIG. 1, it can be seen that the microspheres of Example 5, in which tirzepatide is contained in an amount of 9 wt% or more based on the total weight of the microspheres and a biocompatible polymer is contained at a weight ratio of 9 times or less relative to the tirzepatide content, exhibited excellent sustained-release capability over a long period of time, with no initial burst.

## Claims

1. Microspheres comprising tirzepatide or a pharmaceutically acceptable salt thereof, and a biocompatible polymer, wherein
the tirzepatide or a pharmaceutically acceptable salt thereof is contained in an amount of 9 wt% or more based on the total weight of the microspheres, and
the biocompatible polymer is polylactide-co-glycolide(PLGA) having an intrinsic viscosity of 0.15 dL/g to 1.7 dL/g, and a molar ratio of lactide to glycolide is 50:45 to 55.

2. The microspheres of claim 1, wherein the biocompatible polymer is contained in an amount of 91 wt% or less based on the total weight of the microspheres.

3. The microspheres of claim 1, wherein the release of tirzepatide or a pharmaceutically acceptable salt thereof lasts for more than 20 days.

4. The microspheres of claim 3, wherein tirzepatide or a pharmaceutically acceptable salt thereof contained in the microspheres has an initial burst of 30% or less.

5. The microspheres of claim 1, wherein the microspheres are of an O/W (oil-in-water) type or an W₁/O/W₂ (water-in-oil-in-water) type.

6. A method for preparing microspheres, comprising the steps of:
(a) preparing a dispersed phase by dispersing tirzepatide or a pharmaceutically acceptable salt thereof and a biocompatible polymer in one or more solvents;
(b) adding the prepared dispersed phase to a continuous phase and stirring to form microspheres; and
(c) removing the solvent.

7. The method for preparing microspheres of claim 6, wherein the weight ratio of the biocompatible polymer to the tirzepatide or a pharmaceutically acceptable salt thereof in the step (a) is 9 times or less.

8. The method for preparing microspheres of claim 6, wherein the biocompatible polymer in the step (a) has an intrinsic viscosity of 0.15 dL/g to 1.7 dL/g.

9. The method for preparing microspheres of claim 6, wherein the biocompatible polymer is at least one copolymer selected from polylactide-co-glycolide (PLGA) having an intrinsic viscosity of 0.15 dL/g to 1.7 dL/g and a molar ratio of lactide to glycolide of 50:45 to 55.

10. A method for preparing microspheres, comprising the steps of:
(1) preparing an inner aqueous phase (W₁) by dissolving or dispersing tirzepatide or a pharmaceutically acceptable salt thereof in a solvent;
(2) preparing an oil phase (O) by dissolving a biocompatible polymer in one or more solvents;
(3) preparing a water-in-oil (W₁/O) type emulsion by dispersing the inner aqueous phase (W₁) in the oil phase (O);
(4) forming microspheres by dispersing the water-in-oil (W₁/O) type emulsion into an outer continuous phase (W₂) to prepare a water-in-oil (W₁/O/W₂) type emulsion; and
(5) removing the solvent.

11. The method for preparing microspheres of claim 10, wherein a weight ratio of the biocompatible polymer to tirzepatide or a pharmaceutically acceptable salt thereof in the step (3) is 9 times or less.

12. A pharmaceutical composition for preventing or treating diabetes, obesity, hypertension, hyperlipidemia, non-alcoholic steatohepatitis, metabolic steatohepatitis, cardiovascular disease, or degenerative neurological disease, comprising the microspheres of claim 1 and a pharmaceutically acceptable carrier.

13. The pharmaceutical composition of claim 12, which is a pharmaceutical composition for subcutaneous or intramuscular injection.
